# EUROPÄISCHE PATENTANMELDUNG

(11) **EP 1 870 101 A1**
(43) Veröffentlichungstag der Anmeldung: **26.12.2007**
(21) Anmeldenummer: 07117238.1
(22) Anmeldetag: 21.09.2004
(51) Int. Cl.: A61K 31/46, A61P 43/00

(54) **Aerosolformulierung für die Inhalation enthaltend ein Anticholinergikum**

(30) Priorität: 26.09.2003 DE 10345065
(62) Teilanmeldung aus: 04765441.3
(71) Anmelder: Boehringer Ingelheim Pharma GmbH & Co. KG, 55216 Ingelheim am Rhein (DE); Boehringer Ingelheim International GmbH, 55216 Ingelheim am Rhein (DE)
(72) Erfinder: Schmidt, Friedrich, 55218 Ingelheim (DE); Boeck, Georg, 88471 Laupheim (DE)
(74) Vertreter: Hammann, Heinz

(57) **Zusammenfassung**

Die vorliegende Erfindung betrifft Arzneimittelzubereitungen für die Inhalation enthaltend als alleinigen Wirkstoff eine Verbindung der Formel 1 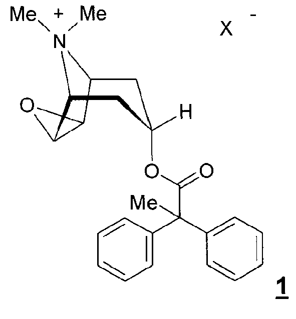 worin
X⁻
ein Anion welches bevorzugt ausgewählt ist aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bedeutet,
als Lösungsmittel Ethanol oder Mischungen aus Ethanol und Wasser, wenigstens eine pharmakologisch verträgliche Säure sowie gegebenenfalls weitere pharmakologisch verträgliche Hilfsstoffe und/oder Komplexbildner.

## Beschreibung

Die vorliegende Erfindung betrifft Arzneimittelzubereitungen für die Inhalation enthaltend als alleinigen Wirkstoff eine Verbindung der Formel **1** 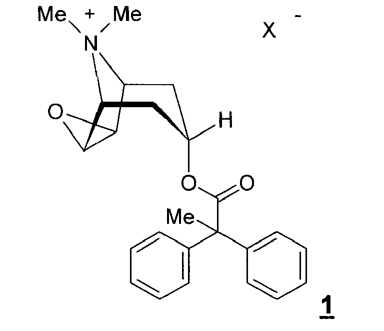 worin
- X⁻: ein Anion welches bevorzugt ausgewählt ist aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bedeutet,
als Lösungsmittel Ethanol oder Mischungen aus Ethanol und Wasser, wenigstens eine pharmakologisch verträgliche Säure sowie gegebenenfalls weitere pharmakologisch verträgliche Hilfsstoffe und/oder Komplexbildner.

Die Verbindungen der Formel **1** sind aus der WO 02/32899 bekannt. Sie besitzen wertvolle pharmakologische Eigenschaften und können als hochwirksame Anticholinergika bei Therapie von Atemwegserkrankungen, insbesondere bei der Therapie entzündlicher und/oder obstruktiver Atemwegserkrankungen, insbesondere bei der Therapie von Asthma oder COPD (chronic obstructive pulmonary disease = chronisch obstruktive Lungenerkrankung) einen therapeutischen Nutzen entfalten.

Die vorliegende Erfindung beschäftigt sich mit inhalativ applizierbaren flüssigen Wirkstoffformulierungen dieser Verbindungen, wobei die erfindungsgemäßen flüssigen Formulierungen hohen Qualitätsstandards genügen müssen. Die erfindungsgemäßen Formulierungen können dabei peroral oder pernasal inhaliert werden. Um eine optimale Wirkstoffverteilung der Wirksubstanzen in der Lunge zu erhalten, bietet sich die Applikation einer flüssigen, auf Treibgase verzichtenden, Formulierung mittels dafür geeigneter Inhalatoren an. Die inhalative Applikation einer solchen Formulierung kann sowohl auf oralem als auch auf nasalem Weg erfolgen. Besonders geeignet sind solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 Mikrolitern, bevorzugt weniger als 50 Mikrolitern, ganz bevorzugt weniger als 20 Mikrolitern Wirkstofflösung mit bevorzugt einem Hub oder zwei Hüben zu einem Aerosol mit einer durchschnittlichen Teilchengröße von weniger als 20 Mikrometern, bevorzugt weniger als 10 Mikrometern, so vernebelt werden können, daß der inhalierbare Anteil des Aerosols bereits der therapeutisch wirksamen Menge entspricht.
Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 "Atomizing Device and Methods" als auch in der WO 97/12687, dort Figuren 6a und 6b und der dazugehörigen Beschreibung, ausführlich beschrieben. In einem solchen Vernebler wird eine Arzneimittellösung mittels hohen Drucks von bis zu 600 bar in ein lungengängiges Aerosol überführt und versprüht. Auf die genannten Referenzen wird im Rahmen der vorliegenden Erfindungsbeschreibung ausdrücklich in Gänze Bezug genommen.
In solchen Inhalatoren werden die Lösungsformulierungen in einem Reservoir gelagert. Dabei ist es notwendig, daß die verwendeten Wirkstoffformulierungen eine ausreichende Lagerstabilität aufweisen und gleichzeitig so beschaffen sind, daß sie dem medizinischen Zweck entsprechend möglichst ohne weitere Manipulation, direkt appliziert werden können. Ferner dürfen sie keine Bestandteile aufweisen, die so mit dem Inhalator wechselwirken können, daß der Inhalator oder die pharmazeutische Qualität der Lösung, respektive des erzeugten Aerosols, Schaden nehmen könnte.

Zur Vernebelung der Lösung wird eine spezielle Düse verwendet, wie sie beispielsweise die WO 94/07607 oder die WO 99/16530 beschreibt. Auf beide wird hiermit ausdrücklich Bezug genommen.

Es ist Aufgabe der vorliegenden Erfindung, eine Formulierung der Verbindung der Formel **1** bereitzustellen, welche den hohen Standards genügt, die notwendig sind, um eine Lösung mittels der eingangs genannten Inhalatoren optimal vernebeln zu können. Die erfindungsgemäßen Wirkstoffformulierungen müssen dabei auch eine ausreichende pharmazeutische Qualität aufweisen, d.h. sie sollten über eine Lagerzeit von einigen Jahren, bevorzugt von mindestens einem Jahr, stärker bevorzugt von zwei Jahren pharmazeutisch stabil sein.
Diese treibgasfreien Lösungsformulierungen müssen ferner mittels eines Inhalators unter Druck vernebelt werden können, wobei die im generierten Aerosol ausgebrachte Masse reproduzierbar innerhalb eines definierten Bereichs liegt.

Im Rahmen der vorliegenden Erfindung gelangen bevorzugt jene Verbindungen der Formel **1** zur Anwendung, in denen das Anion X⁻ ausgewählt ist aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat.

Bevorzugt gelangen die Salze der Formel **1** zur Anwendung, worin X⁻ ein Anion ausgewählt aus der Gruppe Chlorid, Bromid, 4-Toluolsulfonat und Methansulfonat bedeutet.

Erfindungsgemäß besonders bevorzugt im Rahmen der vorliegenden Erfindung sind diejenigen Formulierungen, die die Verbindung der Formel **1**, in der X⁻ für Bromid steht, enthalten.

Bezugnahmen auf die Verbindung der Formel **1** schließen im Rahmen der vorliegenden Erfindung stets alle möglichen amorphen und kristallinen Modifikationen dieser Verbindung mit ein. Bezugnahmen auf die Verbindung der Formel **1** schließen im Rahmen der vorliegenden Erfindung ferner alle möglichen Solvate und Hydrate, die von dieser Verbindung gebildet werden können mit ein.

Eine gegebenenfalls im Rahmen der vorliegenden Erfindung erfolgende Bezugnahme auf die Verbindung **1'** ist als Bezugnahme auf das in den Salzen **1** enthaltene pharmakologisch aktive Kation der nachstehenden Formel anzusehen

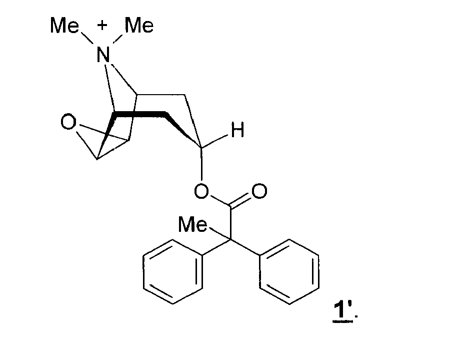

In der erfindungsgemäßen Formulierung liegt die Verbindung **1** gelöst in Ethanol oder in Mischungen aus Ethanol und Wasser vor.

Erfindungsgemäß enthält die Formulierung bevorzugt nur ein einziges Salz der Formel **1.** Allerdings kann die Formulierung auch ein Gemisch verschiedener Salze der Formel **1** enthalten. Formulierungen, die andere Wirkstoffe als jene der Formel **1** enthalten, sind nicht Gegenstand der Erfindung.

Die Konzentration der Verbindung der Formel **1** bezogen auf den Anteil an pharmakologisch wirksamem Kation **1'** in der erfindungsgemäßen Arzneimittelzubereitung liegt erfindungsgemäß bei etwa 4 bis 2000 mg pro 100 ml, bevorzugt bei etwa 8 bis 1600 mg pro 100 ml. Besonders bevorzugt enthalten 100ml der erfindungsgemäßen Formulierungen etwa 80 bis etwa 1360 mg **1'**.

Wird als Verbindung der Formel **1** jene erfindungsgemäß besonders bevorzugte Verbindung eingesetzt, in der X -für das Bromid steht, liegt der Anteil an **1** erfindungsgemäß bei etwa 5 bis 2500 mg pro 100 ml, bevorzugt bei etwa 10 bis 2000 mg pro 100 ml Arzneimittelzubereitung. Besonders bevorzugt enthalten 100ml der erfindungsgemäßen Formulierungen etwa 100 bis 1700 mg **1**.

Erfindungsgemäße Formulierungen enthalten als Lösungsmittel reines Ethanol oder Mischungen aus Ethanol und Wasser. Werden Ethanol-Wasser-Mischungen verwendet, so liegt der prozentuale Massenanteil von Ethanol in diesen Mischungen bevorzugt im Bereich zwischen 5 und 99 % Ethanol, besonders bevorzugt im Bereich von 10 bis 96 % Ethanol. Ganz besonders bevorzugt im Sinne der Erfindung enthalten als Lösungsmittel verwendete Ethanol-Wasser-Mischungen zwischen 50 und 92 %, besonders bevorzugt zwischen 69 und 91 % Ethanol.

Gegebenenfalls können neben Ethanol und Wasser weitere Co-Solventien eingesetzt werden. Erfindungsgemäß bevorzugt gelangt ein weiteres Lösungsmittel allerdings nicht zum Einsatz.

Die erfindungsgemäßen Formulierungen enthalten pharmakologisch verträgliche anorganische oder organische Säuren zur Einstellung des pH-Werts. Der pH-Wert der erfindungsgemäßen Formulierungen liegt erfindungsgemäß bevorzugt in einem Bereich von 2,5 und 6,5, bevorzugt zwischen 3,0 und 5,0, besonders bevorzugt zwischen etwa 3,5 und 4,5.

Beispiele für bevorzugte anorganische Säuren sind ausgewählt aus der Gruppe bestehend aus Salzsäure, Bromwasserstoffsäure, Salpetersäure, Schwefelsäure und Phosphorsäure.
Beispiele für besonders geeignete organische Säuren sind ausgewählt aus der Gruppe bestehend aus Ascorbinsäure, Zitronensäure, Äpfelsäure, Weinsäure, Maleinsäure, Bernsteinsäure, Fumarsäure, Essigsäure, Ameisensäure und Propionsäure. Bevorzugte anorganische Säuren sind Salzsäure und Schwefelsäure, wobei der Salzsäure erfindungsgemäß besondere Bedeutung zukommt. Unter den organischen Säuren sind Ascorbinsäure, Fumarsäure und Zitronensäure bevorzugt, wobei Zitronensäure erfindungsgemäß besonders bevorzugt ist. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe oder Antioxidantien besitzen, wie beispielsweise Zitronensäure oder Ascorbinsäure.
Gegebenenfalls können auch pharmakologisch verträgliche Basen zum genauen Austitrieren des pH-Wertes eingesetzt werden. Als Basen eignen sich beispielsweise Alkalihydroxide und Alkalicarbonate. Bevorzugtes Alkaliion ist Natrium. Werden solche Basen verwendet, ist darauf zu achten, daß auch die daraus resultierenden Salze, die dann in der fertigen Arzneimittelformulierung enthalten sind, mit der oben genannten Säure pharmakologisch akzeptabel sind.

Die erfindungsgemäßen Formulierungen können als weitere Bestandteile Komplexbildner enthalten. Unter Komplexbildner werden im Rahmen der vorliegenden Erfindung Moleküle verstanden, die in der Lage sind Komplexbindungen einzugehen. Bevorzugt sollen durch diese Verbindungen Kationen, besonders bevorzugt metallische Kationen komplexiert werden. Die erfindungsgemäßen Formulierungen enthalten als Komplexbildner bevorzugt Editinsäure (EDTA) oder ein bekanntes Salze davon, z.B. Natrium-EDTA, bzw. Dinatrium-EDTA. Bevorzugt wird Natriumedetat, gegebenenfalls in Form seiner Hydrate, besonders bevorzugt in Form seines Dihydrats eingesetzt.
Werden im Rahmen der erfindungsgemäßen Formulierungen Komplexbildner verwendet, so liegt deren Gehalt bevorzugt in einem Bereich von 1 bis 100 mg pro 100 ml, besonders bevorzugt in einem Bereich von 5 bis 50 mg pro 100 ml der erfindungsgemäßen Formulierung. Vorzugsweise enthalten die erfindungsgemäßen Formulierungen einen Komplexbildner in einer Menge von etwa 6 bis 30 mg pro 100 ml, besonders bevorzugt von etwa 7 bis 20 mg pro 100 ml der erfindungsgemäßen Formulierung.

Analoges wie bereits für Natriumedetat ausgeführt, gilt auch für mögliche, mit EDTA oder seinen Salzen vergleichbare Zusatzstoffe, die komplexbildende Eigenschaften aufweisen und anstelle dessen verwendet werden können, wie beispielsweise Nitrilotriessigsäure und deren Salze.

Der erfindungsgemäßen Formulierung können weitere pharmakologisch verträgliche Hilfsstoffe zugesetzt werden. Unter Hilfs- und Zusatzstoffen wird in diesem Zusammenhang jeder pharmakologisch verträgliche und therapeutisch sinnvolle Stoff verstanden, der kein Wirkstoff ist, aber zusammen mit dem Wirkstoff in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. Stabilisatoren, Antioxidantien und/oder Konservierungsstoffe, die die Verwendungsdauer der fertigen Arzneimittelformulierung verlängern sowie Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit pathogenen Keimen zu schützen. Als Konservierungsstoffe eignen sich die dem Stand der Technik bekannten, insbesondere Benzalkoniumchlorid oder Benzoesäure bzw. Benzoate wie Natriumbenzoat in der aus dem Stand der Technik bekannten Konzentration. Bevorzugt wird der erfindungsgemäßen Formulierung Benzalkoniumchlorid beigemischt. Die Menge des Benzalkoniumchlorids beträgt dabei zwischen 1 und 50 mg pro 100 ml Formulierung, bevorzugt etwa 7 bis 15 mg pro 100 ml, besonders bevorzugt etwa 9 bis 12 mg pro 100 ml der erfindungsgemäßen Formulierung. Erfindungsgemäß besonders bevorzugt sind jedoch Formulierungen, die keine Konservierungsstoffe enthalten.

Bevorzugte Formulierungen enthalten neben Ethanol oder Ethanol-Wasser-Mischungen als Lösungsmittel und den Verbindungen der Formel **1** nur noch Benzalkoniumchlorid, Natriumedetat und die zum Einstellen des pH-Werts notwendige Säure.

Die erfindungsgemäßen Arzneimittelformulierungen mit Verbindungen der Formel **1** werden bevorzugt in einem Inhalator der vorstehend beschriebenen Art verwendet, um daraus die erfindungsgemäßen treibgasfreien Aerosole herzustellen. An dieser Stelle sei deshalb noch einmal ausdrücklich auf die eingangs beschriebenen Patentdokumente verwiesen, auf die hiermit vollinhaltlich Bezug genommen wird.

Wie eingangs geschildert wird eine weiterentwickelte Ausführungsform des bevorzugten Inhalators in der WO 97/12687 (siehe dort insbesondere Figuren 6a und 6b und die diesbezüglichen Beschreibungsteile) offenbart. Dieser Vernebler (Respimat^{®}) kann vorteilhaft zur Erzeugung der erfindungsgemäßen inhalierbaren Aerosole eingesetzt werden. Aufgrund seiner zylinderähnlichen Form und einer handlichen Größe kann dieses Device jederzeit vom Patienten mitgeführt werden. Der Vernebler versprüht ein definiertes Volumen der Arzneimittelformulierung unter Anwendung hoher Drücke durch kleine Düsen, so daß inhalierbare Aerosole entstehen.

Im wesentlichen besteht der bevorzugte Zerstäuber aus einem Gehäuseoberteil, einem Pumpengehäuse, einer Düse, einem Spannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Spannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtungen. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile der o.g. Internationalen Patentanmeldung Bezug genommen. Der Hohlkolben mit Ventilkörper übt auf seiner Hochdruckseite zum Zeitpunkt des Auslösens der Feder einen Druck von 5 bis 60 MPa (etwa 50 bis 600 bar), bevorzugt 10 bis 60 MPa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 10 bis 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-99/16530 offenbart; auf diese Schrift wird hiermit inhaltlich Bezug genommen, insbesondere auf die dort offenbarte Figur 1 und deren Beschreibung.
Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Die flüssige Arzneimittelzubereitung trifft wie bereits erwähnt mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchengrößen des Aerosols liegen bei bis zu 20 Mikrometern, bevorzugt 3 bis 10 Mikrometern.

Das Spannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseoberteil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 6 a/b der WO 97/12687, ist der Vernebler (Respimat®) beschrieben, mit dem die erfindungsgemäßen wäßrigen Aerosolzubereitungen vorteilhaft inhaliert werden können.
Figur 6 a zeigt einen Längsschnitt durch den Zerstäuber bei gespannter Feder, Figur 6 b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseoberteil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

In einer weiteren bevorzugten Ausführungsform gelangt zur Applikation der erfindungsgemäßen Arzneimittelformulierung der vorstehend beschriebene Vernebler zur Anwendung, in dem ein austauschbarer Vorratsbehälter Verwendung findet, der die erfindungsgemäße Arzneimittelformulierung innerhalb eines gas- und flüssigkeitsdichten Behälters enthält, wie er in der WO 99/43571 beschrieben ist. Nachfolgend werden konstruktive Details dieses Behälters näher beschrieben, wobei die in der nachfolgenden Beschreibung angegebenen Bezugszeichen jenen entsprechen, die in der WO 99/43571 offenbart werden. Mit der nachfolgenden Beschreibeung wird mithin vollinhaltlich Bezug auf die Offenbarung der WO 99/43571 genommen.
Dementsprechend wird für die Applikation der erfindungsgemäßen Formulierungen besonders bevorzugt ein gas- und flüssigkeitsdichter Behälter als auswechselbare Kartusche für eine medizinische Flüssigkeit in einem treibgasfreien Zerstäuber verwendet, welcher, wie in der WO 99/43571 offenbart einen Entnahmestutzen in Form eines Hohlkolbens aufweist, wobei der Behälter umfasst
- einen Folienbeutel (11, 21, 31), der an beiden Enden verschlossen ist, wobei mindestens ein Ende durch eine Schweißnaht (13, 23, 32) verschlossen ist, die im wesentlichen quer zur Beutelachse verläuft und der Folienbeutel bei einem Differenzdruck zwischen dem Innenraum des Behälters und seiner Umgebung unterhalb 300 hPa (300 mbar) durch den Außendruck verformbar ist,
- einen formstabilen Flansch (15, 25, 34), der an dem Folienbeutel dicht angebracht ist und der als lösbares Verbindungselement zum Aufstecken des Behälters auf einen Entnahmestutzen (67) ausgebildet ist,
- einen Führungskanal (42, 54) in dem Flansch,
- wobei in dem Führungskanal eine Dichtstelle (56, 64, 74) ausgebildet ist und/oder eine Presspassung (55, 66, 77), die den Entnahmestutzen umschließt
- und eine Entnahmestelle für die Flüssigkeit im Bereich des Führungskanals, in die bei Gebrauch der Hohlkolben sticht, so daß er in die medizinische Flüssigkeit eintaucht.

Wird die erfindungsgemäße Formulierung mittels der vorstehend beschriebenen Technik (Respimat^{®}) vernebelt, sollte die ausgebrachte Masse bei wenigstens 97%, bevorzugt wenigstens 98% aller Betätigungen des Inhalators (Hub oder Hübe) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann auch mittels anderer als der vorstehend beschriebenen Inhalatoren, beispielsweise Jet-Stream-Inhalatoren oder Ultraschallvernebler, vernebelt werden.

Die vorliegende Erfindung betrifft ferner ein Inhalationskit bestehend aus einer der vorstehend beschriebenen erfindungsgemäßen Arzneimittelzubereitungen und einem zur Vernebelung dieser Arzneimittelzubereitung geeigneten Inhalator. Die vorliegende Erfindung betrifft bevorzugt ein Inhalationskit bestehend aus einer der vorstehend beschriebenen erfindungsgemäßen Arzneimittelzubereitungen und dem vorstehend beschriebenen Inhalator Respimat^{®}.

Die nachstehend ausgeführten Formulierungsbeispiele dienen der weitergehenden Erläuterung ohne den Gegenstand der vorliegenden Erfindung auf die exemplarisch dargestellten Zusammensetzungen zu beschränken.

### I. Formulierungsbeispiele

100 ml Arzneimittelzubereitung enthalten:

| Beispiel | **1** (**1**'-Bromid) (mg) | Benzalkonium-chlorid (mg) | Dinatrium-edetat-Dihydrat (mg) | Zitronensäure (mg) | Auf 100 ml mit Ethanol/Wasser Mischung (% m/m) |
|---|---|---|---|---|---|
| 1 | 2000 | 10 | 10 | 3 | 50/50 |
| 2 | 1000 | 5 | - | 3 | 70/30 |
| 3 | 1500 | - | 10 | 5 | 70/30 |
| 4 | 500 | - | 20 | 2 | 70/30 |
| 5 | 150 | - | 10 | 3 | 90/10 |
| 6 | 250 | - | 10 | 2 | 90/10 |
| 7 | 750 | - | - | 4 | 90/10 |
| 8 | 150 | - | - | 3 | 90/10 |
| 9 | 250 | - | - | 4 | 95/5 |
| 10 | 500 | - | - | 3 | 95/5 |
| 11 | 100 | 5 | - | 3 | 95/5 |

Die Darstellung der erfindungsgemäßen Formulierungen erfolgt in Analogie zu im Stand der Technik bekannten Verfahren beispiesweise durch Auflösung der jeweiligen Formulierungsbestandteile im Lösungsmittel Ethanol bzw. Ethanol/Wasser.

## Patentansprüche

1. Arzneimittelzubereitung für die Inhalation enthaltend als alleinigen Wirkstoff eine Verbindung der Formel **1** 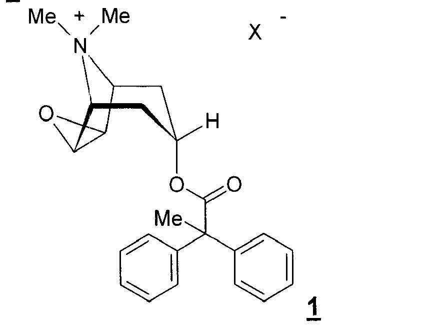 worin
X⁻ ein Anion ausgewählt ist aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat bedeutet,
als Lösungsmittel Ethanol oder Mischungen aus Ethanol und Wasser, wenigstens eine pharmakologisch verträgliche Säure sowie gegebenenfalls weitere pharmakologisch verträgliche Hilfsstoffe und/oder Komplexbildner.

2. Arzneimittelzubereitungen nach Anspruch 1, enthaltend wenigstens eine Verbindung der Formel **1** worin X- ausgewählt ist aus der Gruppe bestehend aus Chlorid, Bromid, 4-Toluolsulfonat und Methansulfonat.

3. Arzneimittelzubereitungen nach Anspruch 1 oder 2, die Mischungen aus Ethanol und Wasser als Lösungsmittel enthalten, wobei der Massenanteil von Ethanol in diesen Mischungen im Bereich von 5 bis 99 % liegt.

4. Arzneimittelzubereitungen nach einem der Ansprüchen 1 bis 3, die Mischungen aus Ethanol und Wasser als Lösungsmittel enthalten, wobei der Massenanteil von Ethanol in diesen Mischungen im Bereich von 10 bis 96 % liegt.

5. Arzneimittelzubereitungen nach einem der Ansprüche 1 bis 4, worin die pharmakologisch verträgliche Säure ausgewählt ist aus den anorganischen Säuren Salzsäure, Bromwasserstoffsäure, Salpetersäure, Schwefelsäure und Phosphorsäure oder aus den organischen Säuren Ascorbinsäure, Zitronensäure, Äpfelsäure, Weinsäure, Maleinsäure, Bernsteinsäure, Fumarsäure, Essigsäure, Ameisensäure und Propionsäure.

6. Arzneimittelzubereitungen nach einem der Ansprüche 1 bis 5, **gekennzeichnet durch** einen pH von 2,5 bis 6,5.

7. Arzneimittelzubereitung nach einem der Ansprüche 1 bis 6, **dadurch gekennzeichnet, dass** der Gehalt an **1'** etwa 4 bis 2000 mg pro 100 ml Lösung beträgt.

8. Arzneimittelzubereitung nach einem der Ansprüche 1 bis 7, **dadurch gekennzeichnet, daß** sie als weiteren Bestandteil einen Komplexbidner enthalten.

9. Arzneimittelzubereitung nach einem der Ansprüche 1 bis 8, **dadurch gekennzeichnet daß** der Gehalt an Komplexbildner 1 bis 100 mg pro 100 ml Lösung beträgt.

10. Arzneimittelzubereitung nach einem der Ansprüche 1 bis 9, **dadurch gekennzeichnet, daß** sie als Hilfsstoff Benzalkoniumchlorid enthalten.

11. Arzneimittelzubereitung nach Anspruch 10, **dadurch gekennzeichnet daß** der Gehalt an Benzalkoniumchlorid 1 bis 50 mg pro 100 ml Lösung beträgt.

12. Verwendung einer Arzneimittelzubereitung nach einem der Ansprüche 1 bis 11 zur Herstellung eines Arzneimittels zur Behandlung von Atemwegserkrankungen.

13. Verfahren zur inhalativen Anwendung einer Arzneimittelzubereitung nach einem der Ansprüche 1 bis 12 auf oralem oder nasalem Weg.

14. Verwendung einer Arzneimittelzubereitung gemäß einem der Ansprüche 1 bis 13 zur Vernebelung in einem Inhalator gemäß der WO 91/14468 oder einem wie in den Figuren 6a und 6b der WO 97/12687 beschriebenen Inhalator.

15. Inhalationskit bestehend aus einer Arzneimittelzubereitung nach einem der Ansprüche 1 bis 14 und einem zur Vernebelung dieser Arzneimittelzubereitung geeigneten Inhalator.

16. Inhalationskit nach Anspruch 15, wobei der Inhalator der Respimat^{®} ist.
